# EUROPEAN PATENT SPECIFICATION

(11) **EP 2 156 781 B1**
(45) Date of publication and mention of the grant of the patent: **31.07.2013**
(21) Application number: 08764017.3
(22) Date of filing: 04.06.2008
(51) Int. Cl.: A61B 1/00, A61B 5/07, G06F 19/00

(54) **CAPSULE TYPE ENDOSCOPE SYSTEM, AND PROGRAM AND METHOD USED BY THE SYSTEM**
KAPSELARTIGES ENDOSKOPSYSTEM SOWIE VOM SYSTEM BENUTZTES PROGRAMM UND VERFAHREN
SYSTEME ENDOSCOPIQUE DE TYPE A CAPSULE, ET PROGRAMME ET PROCEDE UTULISE PAR LE SYSTEME

(30) Priority: 06.06.2007 JP 2007150675
(43) Date of publication of application: 24.02.2010
(73) Proprietor: Olympus Medical Systems Corp., Tokyo 151-0072 (JP)
(72) Inventor: FUJITA, Manabu, Tokyo 151-0072 (JP)
(74) Representative: von Hellfeld, Axel
(86) International application number: PCT/JP2008/001420
(87) International publication number: WO 2008/149549

(56) References cited:
- WO-A1-2007/074712
- WO-A2-2005/062715
- WO-A2-2006/003650
- JP-A- 2005 296 186
- JP-A- 2006 020 971
- JP-A- 2006 061 470
- JP-A- 2006 130 322
- JP-A- 2006 239 309
- JP-A- 2006 288 542
- US-A1- 2005 171 418

## Description

### Technical Field

The present invention relates to a technique for managing a data transmission performed for utilizing, in an information processing apparatus, image data transmitted from a capsule-type endoscope to a receiver.

### Background Art

Endoscopes have frequently been used to observe the inside of a human body in recent years. An endoscope includes an imaging unit so that the image data obtained by imaging inside of a human body by the imaging unit is transmitted to an external apparatus and an image is displayed. A physician observes the displayed image and performs diagnosis, operations, et cetera. The transmission of the image data from the endoscope to an external apparatus is performed by way of a cable or by using radio communications. The latter type of endoscope is also called a wireless endoscope. To use it, a receiver is required to receive the image data transmitted by way of the radio communication.

As an example, patent document 1 notes a surgery apparatus including one wireless endoscope and two transmitting and receiving apparatuses. The two transmitting and receiving apparatuses severally receive image data from the wireless endoscope. Even if a reception failure occurs in one of the transmitting and receiving apparatuses, the image data is transferred thereto after being received at the other transmitting and receiving apparatus from the wireless endoscope, and therefore the reception condition can be recovered to a good condition.

Meanwhile, the wireless endoscopes also include a type called a capsule-type endoscope, used in combination with a portable receiver equipped on an examinee. A capsule-type endoscope includes an imaging unit and a wireless transmission unit for transmitting image data obtained by imaging to an external apparatus by way of a wireless communication. The capsule-type endoscope is used as follows.

First, the examinee swallows the capsule-type endoscope. The capsule-type endoscope proceeds through the examinee's body by means of peristaltic movement of the digestive canal for about eight hours, and is eventually ejected from the body. While the capsule-type endoscope is within the body, the image data obtained by the imaging unit performing imaging operation is transmitted by the wireless transmission unit and is received by the receiver. The receiver stores the received image data in a built-in storage unit or in a removable storage medium. If the imaging unit is configured to image, for example, two times per second, pieces of image data equivalent to approximately 60,000 images are accumulated in the receiver in about eight hours.

The image data accumulated in the receiver is transmitted to an information processing apparatus such as a work station after the capsule-type endoscope is ejected out of the examinee's body. The transmission takes a relatively long period of time. As an example, if approximately 60, 000 images are accumulated in the receiver throughout one examination as in the above example, it sometimes takes tens of minutes to transmit the vast amount of image data. Those pieces of image data transmitted to the information processing apparatus are displayed on a display apparatus as images so as to be observed by a physician or the like.

As the popularization of the capsule-type endoscope progresses, it is predicted that in more cases, a plurality of examinees will use the capsule-type endoscopes simultaneously or successively in a single hospital or inspection agency. This prompts a need to solve problems particular to the existence of a plurality of receivers.

For example, one of the problems is a possibility of misidentification of an examinee when equipping the examinee with a receiver. Patent document 2 notes a receiver (an external device) importing the identification information specific to an examinee and storing and displaying it in order to prevent such misidentification. An example of the identification information is information related to the name, birth date, sex, et cetera. A physician or the like collates the identification information displayed on the receiver with the examinee, thereby enabling the prevention of misidentifying the examinee as another examinee. The identification information is read from the work station and imported into the receiver. To import the identification information, a removable portable storage medium may be used or the receiver may be connected to the work station via a cradle.

The problems specific to the existence of a plurality of receivers are not limited to the misidentification of examinees. With respect to managing transmissions of respective pieces of image data from a plurality of receivers, a device for improvement in efficiency, operability, or convenience is required.

As an example, when an N number of examinees are simultaneously examined by using capsule-type endoscopes, the operator of a capsule-type endoscope system carries out processes for transmitting respective pieces of image data from N receivers to an information processing apparatus such as a work station after finishing the examination of the N examinees. These processes may be, for example, connecting one receiver to the information processing apparatus, performing a transmission from the receiver, and repeating a step of switching the receiver connected to the information processing apparatus to the next receiver upon completing the present transmission so as to perform the transmission from the receiver that was switched to.

Such a method, however, requires operator involvement and manual work every time the receivers are replaced. Further, if an operation and management method in which an operator is constantly standing by for the requirements is adopted, the operator is unnecessarily detained because the transmission requires a relatively a long period of time as described above, and therefore the work efficiency is not high. Further, it might be possible for an operator to replace the receivers every time the transmission from one receiver is completed while the operator is engaged in other work. In this case, however, the operator is required to interrupt this other work again and again.

Accordingly, when a receiver is configured to accumulate image data in a removable storage medium, a contrivance is made to reduce the frequency of operator intervention by processing M storage media taken out of M receivers en masse, so as to improve the work efficiency.

For example, it is possible to adopt a method in which a storage media reading apparatus (reader) having four slots is connected to an information processing apparatus so as to be used for the transmission of image data; and in this case, M= 4. In this method, an operator takes out the storage media respectively from the four receivers, inserts the four storage media into the four slots, respectively, and instructs the information processing apparatus to start transmission, the mere operation of which causes the image data for the respective four examinees to be automatically and sequentially transmitted to the information processing apparatus. Therefore, the operator is relieved from the burden of waiting in front of the information processing apparatus until the transmission of the image data for the four examinees is completed, enabling her/him to be engaged in other work without being interrupted.

If the receiver is configured to accumulate image data in a built-in storage unit in place of a removable storage medium, however, the aforementioned method cannot be adopted, and therefore the work efficiency and convenience of the operator is not sufficiently supported.

Further, if a plurality of examinees has to be examined one after another, there is sometimes a need to reuse the storage medium and/or receiver as soon as the transmission of image data is completed. For example, in the above-mentioned example in which the storage media reading apparatus having four slots is used, there is sometimes a need to take a first storage medium from the reading apparatus and to reuse the first storage medium when the transmission of the image data from the first storage medium is completed. The appearance of the storage medium, however, does not indicate which storage medium corresponds to which examinee, and therefore the operator has not conventionally been able to easily determine which storage medium is to be taken out from the reading apparatus for reuse. That is, the storage media have not been enabled to be efficiently reused through a simple operation, which has sometimes been inconvenient.
Patent document 1: Laid-Open Japanese Patent Application Publication No. 2001-275950
Patent document 2: Laid-Open Japanese Patent Application Publication No. 2005-296186

### Disclosure of Invention

### Problems to be Solved by the Invention

As described above, certain factors exist that undermine the work efficiency of an operator and the reuse efficiency of a receiver related to the transmission of image data from the receiver to an information processing apparatus in the case where there exist a plurality of receivers to receive the image data from capsule-type endoscopes. That is, there is room in the conventional system for improving the efficiency, operability, or convenience.

Thus, the present invention aims at improving the efficiency, operability, or convenience related to the transmission of image data to an information processing apparatus and from a plurality of receivers which have received the image data from capsule-type endoscopes.

### Means for Solving the Problems

A capsule-type endoscope system according to the present invention includes a plurality of receivers for receiving, from a capsule-type endoscope by way of a wireless communication, image data of an observation image of an inside of a body of an examinee imaged by the capsule-type endoscope; a plurality of mounting units for respectively mounting the plurality of receivers; and an information processing apparatus connected to the plurality of receivers by way of the mounting units.

Each of the plurality of receivers includes a first storage unit and a first display unit. The first storage unit stores examinee identification information identifying the examinee and the received image data. The first display unit displays the examinee identification information or examinee information about the examinee who is associated with the examinee identification information.

Further, the information processing apparatus includes a management unit for receiving the examinee identification information from each of the plurality of receivers by way of the mounting unit and for managing a correspondence relation between receiver identification information identifying the receiver or mounting unit identification information identifying the mounting unit on which the receiver is mounted and the received examinee identification information; a second storage unit; and a second display unit.

The image data is transmitted to the information processing apparatus by way of the mounting unit and is stored in the second storage unit. The second display unit displays the correspondence relation by displaying at least a part of the examinee identification information displayed on the first display unit or at least a part of the examinee information displayed on the first display unit. The second display unit further displays a progress of the transmission of the image data corresponding to each of the plurality of receivers.

Depending on an embodiment, the plurality of mounting units may be a plurality of slots provided at one cradle device or may be slots provided at each of a plurality of cradle devices.

In the latter case, the mounting unit identification information may be either port identification information identifying a plurality of ports which are comprised in the information processing apparatus and to which the plurality of cradle devices are respectively connectable, or cradle device identification information identifying the plurality of cradle devices.

A program according to the present invention is a program for causing the information processing apparatus in the capsule-type endoscope system to perform the above described management and display of the correspondence relation and the above described display of the progress. A method according to the present invention is a method executed by the information processing apparatus in accordance with the program.

### Effect of the Invention

The present invention enables a plurality of receivers including a first storage unit to be connected en masse to an information processing apparatus by way of a plurality of mounting units. It is therefore possible to reduce the time an operator is detained or the number of times the operator has to interrupt his/her other work.

The present invention is also contrived such that the progress of the transmission is displayed on the second display unit, and such that the correspondence relation between the mounting unit identification information or receiver identification information and the examinee identification information includes the examinee identification information or examinee information. Therefore, an operator can recognize the progress of transmission from each of the receivers only by comparing the first and second display units. That is, the operator can easily discern a receiver that is ready for reuse at the completion of transmission, and the receiver can be reused efficiently without requiring a complex operation.

### Brief Description of Drawings

Fig. 1 is the external appearance diagram of devices constituting a capsule-type endoscope system according to a first embodiment of the present invention;
Fig. 2 is a block diagram showing the configuration of a capsule-type endoscope system according to the first embodiment of the present invention;
Fig. 3 is a diagram exemplifying a slot correspondence table according to the first embodiment of the present invention;
Fig. 4 is a flow chart exemplifying the utilization of a capsule-type endoscope system according to the first embodiment of the present invention;
Fig. 5 is a diagram exemplifying a screen displayed when initialization is performed in the first embodiment of the present invention;
Fig. 6 is a diagram exemplifying the screen of an observation apparatus when two receivers are respectively mounted on cradles after the completion of examinations in the first embodiment of the present invention;
Fig. 7 is a diagram exemplifying the screen of an observation apparatus when image data is transferred from a receiver in the first embodiment of the present invention;
Fig. 8 is a block diagram showing the configuration of a capsule-type endoscope system according to a second embodiment of the present invention;
Fig. 9 is a diagram exemplifying the screen of an observation apparatus when image data is transferred in the second embodiment of the present invention;
Fig. 10 is a perspective view of a cradle used in a third embodiment of the present invention;
Fig. 11 is a block diagram showing the configuration of a capsule-type endoscope system according to the third embodiment of the present invention; and
Fig. 12 is a diagram exemplifying a slot correspondence table according to the third embodiment of the present invention.

### Explanation of the Signs

101, 102 and 110 Cradle
103 and 104 Charger circuit
111 to 114 Slot
120 Power supply
121 Charger circuit
122 Control circuit
201 and 202 Receiver
203, 204 Display unit, LCD
205 and 206 Patient information storage unit
205b and 206b Patient information/receiver ID storage unit
207 and 208 Control circuit
209 and 210 Battery
301 Observation apparatus
302 ROM
303 RAM
304 CPU
305 Video controller
306 LCD
307 Hard disk
308 Serial I/F
309 Keyboard
310 Bus
311 to 313 Communication I/F
401 and 402 Slot correspondence table
411 to 413 Port address
421 and 422 Patient information
501 to 506 Screen
601 Power supply

### Best Mode for Carrying Out the Invention

The following is a description, in detail, of the embodiments of the present invention by referring to the drawings.

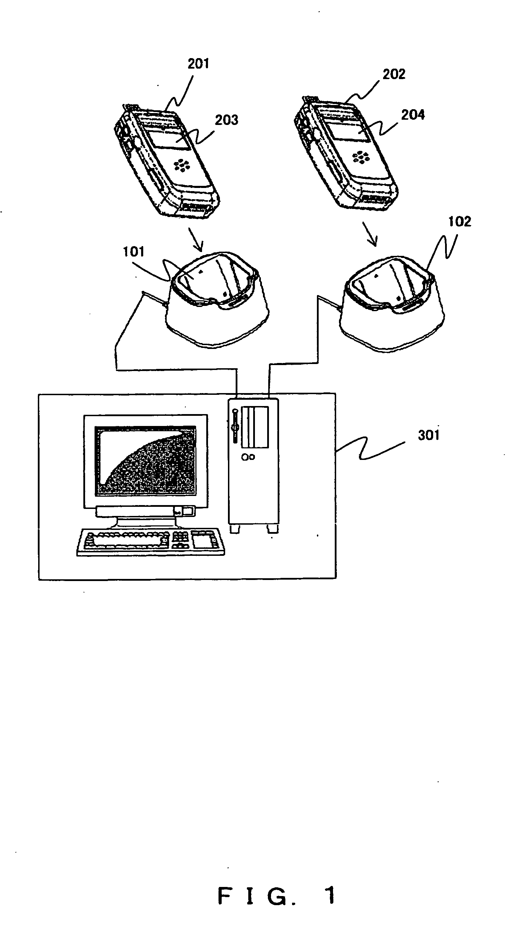

Fig. 1 is the external appearance diagram of devices constituting a capsule-type endoscope system according to a first embodiment of the present invention. The capsule-type endoscope system shown in Fig. 1 includes two receivers 201 and 202, and an observation apparatus 301, with the receivers 201 and 202 enabled to be respectively connected via cradles 101 and 102 to the observation apparatus 301. Fig. 1 shows, for simplicity, two receivers and two cradles. However, the number of receivers and cradles may be three or more. In addition, the number of receivers and cradles may not be the same.

The receiver 201 is a portable device attached to a patient who is not shown. Note that the following embodiments use the word "patient" as having the same meaning as a general examinee. While a capsule-type endoscope that is not shown stays inside of a patient's body, the receiver 201 receives image data obtained by imaging inside of the patient's body from the capsule-type endoscope by way of a wireless communication and stores the image data. An external surface of the receiver 201 is equipped with a display unit 203, of which the display examples are described later. The configuration of the receiver 202 is similar to that of the receiver 201. The receiver 202 is also equipped with a display unit 204 on its external surface.

The observation apparatus 301 is an information processing apparatus such as a general purpose workstation. The cradle 101 is connected to the observation apparatus 301 by way of a cable. Mounting the receiver 201 on the cradle 101 enables a data transmission between the receiver 201 and the observation apparatus 301 via the cradle 101. Similarly, the cradle 102 is connected to the observation apparatus 301 by way of a cable. Then, mounting the receiver 202 on the cradle 102 enables a data transmission between the receiver 202 and the observation apparatus 301 via the cradle 102.

Fig. 1 shows the case of the receiver 201 being mounted on the cradle 101 and the receiver 202 being mounted on the cradle 102. However, the correspondence between a receiver and a cradle is not fixed, which will be described later. It is arbitrary which receiver is mounted on which cradle.

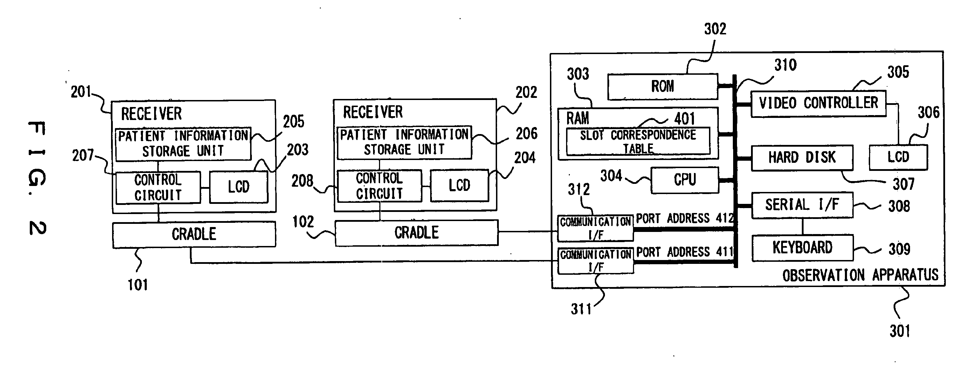

Fig. 2 is a block diagram showing the configuration of a capsule-type endoscope system according to the first embodiment of the present invention. Similarly to Fig. 1, Fig. 2 also shows the case of the receivers 201 and 202 being respectively mounted on the cradles 101 and 102.

As shown in Fig. 2, in the first embodiment, the display unit 203 of the receiver 201 is an LCD (liquid crystal display) 203. The LCD 203 corresponds to the above-noted first display unit. The display unit 203 is not limited to the LCD. Anything suitable for a portable device, such as an EL (electroluminescence) element, may be used. The receiver 201 also includes a wireless reception unit (not shown) for receiving image data transmitted from a capsule-type endoscope by way of a wireless communication.

The receiver 201 further includes a patient information storage unit 205 for storing information such as the image data received from the capsule-type endoscope and a patient ID described later, and includes a control circuit 207 for controlling various processes.

The patient information storage unit 205 corresponds to the above-noted first storage unit and is a built-in storage unit which is implemented by, for example, a hard disk, a flash memory, or the like. The control circuit 207 controls various processes; for example, the following (a) through (c).
(a) The process for writing, to the patient information storage unit 205, data transmitted from the observation apparatus 301 by way of the cradle 101;
(b) The process for reading data from the patient information storage unit 205 and for transmitting it to the observation apparatus 301 by way of the cradle 101; and
(c) The process for reading necessary data from the patient information storage unit 205 and displaying it in the LCD 203.

As with the receiver 201, the receiver 202 also includes an LCD 204, a wireless communication unit (not shown), a patient information storage unit 206, and a control circuit 208.

The observation apparatus 301 includes a ROM (read only memory) 302, a RAM (random access memory) 303, a CPU (central processing unit) 304, a video controller 305, a hard disk 307, a serial I/F (interface) 308, a communication I/F 311, and a communication I/F 312, all of which are connected to one another via a bus 310. Further, an LCD 306 is connected to the video controller 305, and a keyboard 309 is connected to the serial I/F 308. Also, a mouse (not shown) or the like may be connected to the serial I/F 308.

The ROM 302 or the hard disk 307 stores a program that is loaded onto the RAM 303 and executed by the CPU 304. By this, various controls described later are implemented. Further, the program may be stored in a computer readable portable storage medium. Connecting the drive apparatus (not shown) of the portable storage medium to the observation apparatus 301 enables the observation apparatus 301 to read the program from the portable storage medium and to copy it in the hard disk 307.

The video controller 305 controls the display on the LCD 306 in accordance with the instruction from the CPU 304. An example display of the LCD 306 is described later. The LCD 306 corresponds to the above-noted second display unit.

The hard disk 307 stores image data transmitted from the receiver 201 or 202 by way of the cradle 101 or 102. The hard disk 307 corresponds to the above-noted second storage unit.

An instruction input by an operator from the keyboard 309 is reported to the CPU 304 by way of the serial I/F 308.

Port addresses 411 and 412 are assigned respectively to the communication I/Fs 311 and 312. Further, the communication I/Fs 311 and 312 are respectively connected to the cradles 101 and 102 by way of cables.

When the receiver 201 or 202 which was dismounted from the patient and retrieved after completion of the examination is mounted on the cradle 101 or 102, the communication I/F 311 or 312 detects this event. With the detection being used as a trigger, a record is added to a slot correspondence table 401 retained in the RAM 303. In contrast, when the receiver 201 or 202 is detached from the cradle 101 or 102, the communication I/F 311 or 312 also detects this event and, with the detection being used as a trigger, a record is eliminated from the slot correspondence table 401. The details of the slot correspondence table 401 are described later. In the present embodiment, the above-noted management unit is realized by the communication I/Fs 311 and 312, the CPU 304, and the RAM 303.

Further, the above-noted mounting units correspond to the respective slots of the cradles 101 and 102 according to the present embodiment. The slot is a concave part provided on the cradle so as to allow a receiver to be mounted as shown in Fig. 1. The receiver and the cradle are electrically interconnected by inserting the receiver into the slot. Two receivers can be connected together to the observation apparatus 301 by means of the cradles 101 and 102 respectively having slots corresponding to mounting units and via cables connecting the cradles 101 and 102 to the observation apparatus 301.

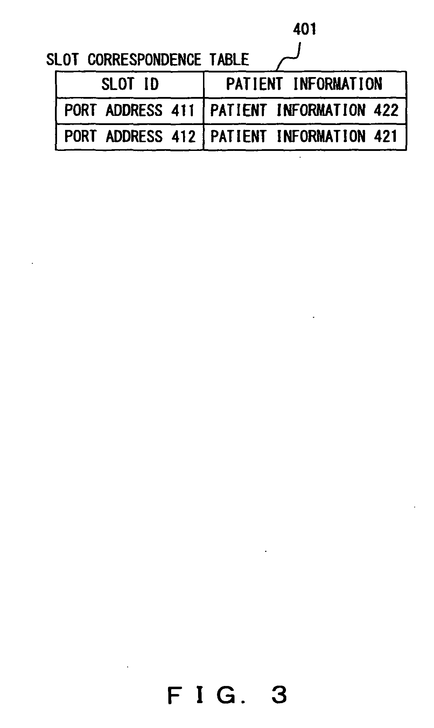

Next is a description of the slot correspondence table by referring to Fig. 3. Fig. 3 is a diagram exemplifying a slot correspondence table according to the first embodiment of the present invention. The slot correspondence table 401 shown in Fig. 3 is a table for managing the correspondence relation between information identifying a slot and information identifying a patient to whom the receiver inserted into the slot was attached.

In the example shown in Fig. 3, the information identifying a slot is called a "slot ID". Identifying a slot makes it possible to indirectly identify a receiver inserted in the slot. Further, the slot correspondence table 401 shown in Fig. 3 may include information associated with a patient ID, such as the sex and/or birth date of a patient, in addition to the patient ID that is the information identifying the patient. The patient ID and information such as the sex and/or birth date are hereinafter collectively called "patient information".

The slot correspondence table 401 utilizes the port address of the observation apparatus 301 as a slot ID. The reason for this is as follows.

As shown in Fig. 1, there is only one slot in a single cradle in the present embodiment. Further, one cradle corresponds to one communication I/F and a unique port address is assigned to the communication I/F, as is clear from Fig. 2. Further, the present embodiment premises that, once cradles are connected to the observation apparatus 301, a capsule-type endoscope system is in principle operated continually without changing the manner of connecting the cables between the cradles and the observation apparatus 301. Therefore, it is possible to uniquely identify a slot on the basis of the port address. This is why the example of Fig. 3 utilizes a port address as a slot ID.

Specifically, the first record in the slot correspondence table 401 expresses that the port address 411 corresponds to the patient information 422, and the second record expresses that the port address 412 corresponds to the patient information 421. As shown in Fig. 2, the port addresses 411 and 412 are respectively assigned to the communication I/Fs 311 and 312. Further, the communication I/Fs 311 and 312 are respectively connected to the cradles 101 and 102 and it is premised that the connection is fixed as described above and that the correspondence relation between the communication I/F and the cradle will not be changed.

Therefore, the slot correspondence table 401 shown in Fig. 3 indicates the following (a) and (b).
(a) A receiver accumulating the image data of a patient indicated by the patient information 422 is mounted on the cradle 101.
(b) A receiver accumulating the image data of a patient indicated by the patient information 421 is mounted on the cradle 102.

Further, the slot correspondence table 401 is stored in the RAM 303, and a record is created or deleted using as a trigger the establishment or elimination of the connection between the cradle and the receiver, as described by referring to Fig. 2.

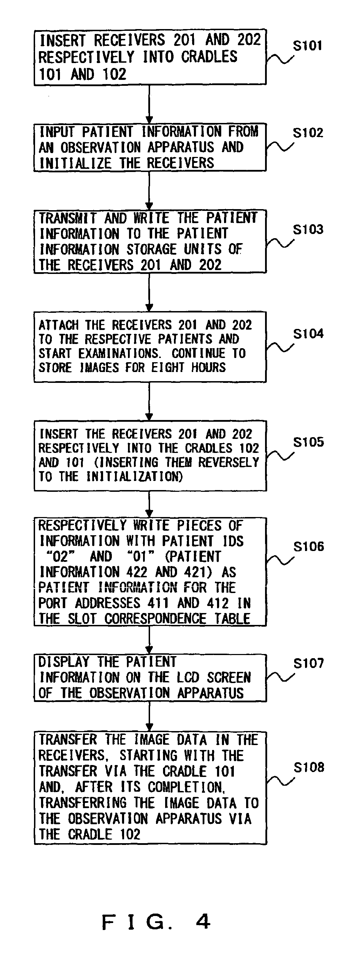

Next is a description of the process flow and screen examples in a utilization example of a capsule-type endoscope system according to the first embodiment of the present invention by referring to Figs. 4 through 7.

Fig. 4 is a flow chart exemplifying the utilization of a capsule-type endoscope system according to the first embodiment of the present invention. Fig. 4 exemplifies the case of simultaneously examining two patients whose patient IDs are "01" and "02".

In step S101, the operator inserts the receivers 201 and 202 respectively into the slots of the cradles 101 and 102. That is, the operator mounts the receivers 201 and 202 onto the respective cradles 101 and 102. This consequently enables a data transmission between the receiver 201 and the observation apparatus 301, and also enables a data transmission between the receiver 202 and the observation apparatus 301.

Then in step S102, the operator, from the keyboard 309 of the observation apparatus 301, inputs the patient information of the two patients, who are examination targets, and also gives an instruction to initialize the receivers 201 and 202 mounted on the cradles 101 and 102.

To describe the details of the process of step S102, first, the following two premises are given.

The first premise is that the operator or another person predetermines the following (a) and (b).
(a) The receiver 201 mounted on the cradle 101 shall be attached on a patient whose patient ID is "01"; and
(b) The receiver 202 mounted on the cradle 102 shall be attached on a patient whose patient ID is "02".

The second premise is that a database storing various pieces of information related to patients exists in the hard disk 307 of the observation apparatus 301 shown in Fig. 2 or in a storage apparatus (not shown) connected to the observation apparatus 301 by way of a network. Therefore, the operator is enabled to call up information such as name, sex, birth date, medical history, et cetera, related to a patient ID just by inputting, for example, the patient ID from the keyboard 309 in step S102.

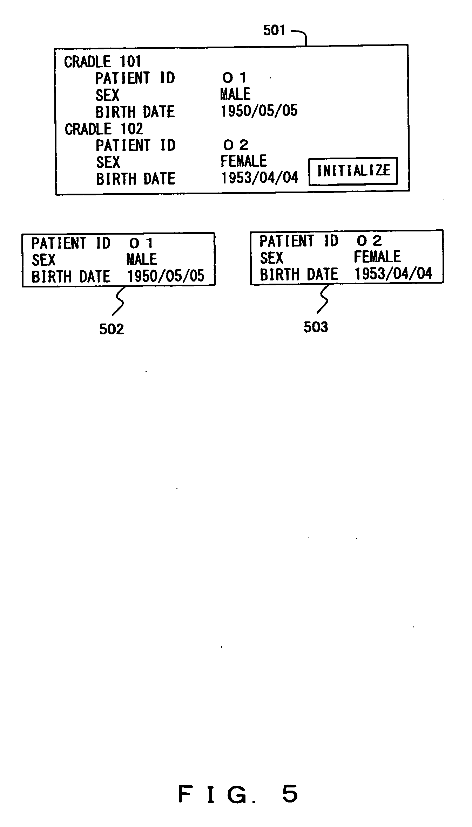

The process of step S102 under these premises is further specifically described by referring to Fig. 5, which shows a screen example of step S102.

In accordance with the first premise, the operator inputs a value "01" as a patient ID corresponding to the cradle 101, and inputs a value "02" as a patient ID corresponding to the cradle 102. This causes the call-up, from the database, of sexes and birth dates from among the patient information of the two patients, on the basis of the second premise. As shown in Fig. 5, the called-up pieces of patient information are related to cradle IDs for identifying the cradles written as "cradle 101" and "cradle 102", respectively, and are displayed on the screen 501 of the LCD 306 of the observation apparatus 301.

Further, the screen 501 also displays a button with "initialize" written on it for instructing the receivers 201 and 202 to initialize themselves. The operator uses, for example, a mouse to click on the "initialize" button, thereby making it possible to instruct, from the observation apparatus 301 by way of the cradles 101 and 102, the receivers 201 and 202 to initialize themselves.

Next, the description will be about Fig. 4 again. In step S103 following step S102, the observation apparatus 301, in accordance with the operator's input in step S102, transmits respective pieces of the patient information of the two patients to the receivers 201 and 202 and instructs the receivers 201 and 202 to perform initialization. The patient information transmitted in step S103 according to the present embodiment is a patient ID, sex, and birth date.

Then, the control circuit 207 of the receiver 201 performs a control for initializing the patient information storage unit 205 in accordance with the instruction. The initialization of the patient information storage unit 205 includes the processes of the following (a) through (c).
(a) The process for writing, into the patient information storage unit 205, the patient information transmitted from the observation apparatus 301;
(b) The process for displaying the written patient information on the LCD 203; and
(c) The process for erasing any remaining image data from the previous use of the receiver 201.

Likewise, the control circuit 208 of the receiver 202 also initializes the patient information storage unit 206.

As a result, as shown in Fig. 5, the screen 502 of the LCD 203 of the receiver 201 displays the same patient ID, sex and birth date as those transmitted from the observation apparatus 301. Likewise, the screen 503 of the LCD 204 of the receiver 202 also displays the same patient ID, sex and birth date as those transmitted from the observation apparatus 301. The operator is enabled to confirm whether or not the receivers 201 and 202 have been correctly initialized, by comparing the screens 501, 502 and 503. Note that the slot correspondence table 401 in Fig. 3 is not used during the processes up to step S103 and no record exists therein.

Having confirmed that the receivers 201 and 202 have been correctly initialized in step S103, the operator, a nurse, or the like attaches the receiver 201 to a patient whose patient ID is "01" and attaches the receiver 202 to a patient whose patient ID is "02", in step S104. Then, these patients respectively swallow a capsule-type endoscope, and the examinations start. As described above, the capsule-type endoscope moves in the interior of the body of the patient for about eight hours. In the meantime, the inside of the patient's body is imaged by the capsule-type endoscope and the image data obtained thereby is transmitted by way of wireless communications. The receiver 201 receives the image data and stores it in the patient information storage unit 205 under the control of the control circuit 207. Likewise, the receiver 202 also receives image data and stores it in the patient information storage unit 206 under the control of the control circuit 208.

After the capsule-type endoscopes are ejected out of the patients' bodies, the receivers 201 and 202 are detached from the patients and the process proceeds to step S105, in which the operator mounts the two receivers onto the two cradles. In this event, each of the receivers can be arbitrarily mounted on either of the cradles and therefore the operator need not be concerned about the combination of receiver and cradle.

The following description is provided by exemplifying the case in which the two receivers are mounted onto the cradles in a reverse order to the initialization. That is, the operator inserts the receiver 201 into the slot of the cradle 102, and inserts the receiver 202 into the slot of the cradle 101, in step S105 shown in Fig. 4.

Then, two records are created in the slot correspondence table 401 on the RAM 303 of the observation apparatus 301 in step S106. As a result, the slot correspondence table 401 turns into a state as shown in Fig. 3. Note that the patient information 421 according to the present embodiment is the information that includes "01" as the patient ID, "male" as the sex and "1950/05/05" as the birth date, as shown in Fig. 5. Meanwhile, the patient information 422 according to the present embodiment is the information that includes "02" as the patient ID, "female" as the sex and "1953/04/04" as the birth date.

That is, when the receiver 202 is inserted into the slot of the cradle 101 in step S105, the communication I/F 311 connected to the cradle 101 detects the insertion and reports the insertion to the CPU 304 in step S106. Using this report as a trigger, the CPU 304 instructs the receiver 202 mounted on the cradle 101 to transmit the patient information stored in the patient information storage unit 206. Then, the receiver 202 transmits the patient information 422 to the observation apparatus 301 in accordance with the instruction. The CPU 304 associates the patient information 422 received at the communication I/F 311 with the port address 411 of the communication I/F 311 to generate a new record in the slot correspondence table 401.

Likewise, when the receiver 201 is inserted into the slot of the cradle 102 in step S105, a record in which the port address 412 and patient information 421 are associated with each other is generated in the slot correspondence table 401 in step S106. As a result, the slot correspondence table 401 turns into the state as shown in Fig. 3.

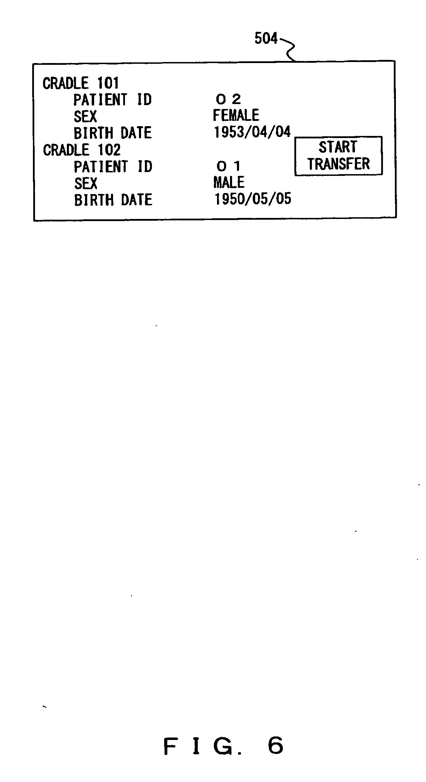

Then, the CPU 304 performs a control for displaying the patient information on the LCD 306 of the observation apparatus 301 in step S107. Fig. 6 is a diagram exemplifying the screen 504 of the LCD 306 in step S107. In accordance with the contents of the slot correspondence table 401, on the screen 504, the patient information 422 is displayed associated with the cradle ID "cradle 101" and the patient information 421 is displayed associated with the cradle ID "cradle 102".

Here, comparing the slot correspondence table 401 shown in Fig. 3 with Fig. 6, the port addresses 411 and 412 are used as the slot ID identifying a slot in the slot correspondence table 401, while the cradle IDs are used in the screen 504 of Fig. 6.

As described above, in the first embodiment, since there is a 1: 1: 1 correspondence between a port address, a cradle, and a slot, it is possible to use a port address or a cradle ID in order to identify a slot. The first embodiment is an example in which two kinds of information for identifying a slot are used.

The operator can visually identify the cradles 101 and 102 if, for example, labels on which the respective cradle IDs are written are pre-attached on the cradles 101 and 102, respectively. In the meantime, a port address is a value managed within the observation apparatus 301 and therefore it is not suitable for an application in which the operator discerns a port at a glance. Accordingly, in the first embodiment, they are used in different ways, i.e., a port address is used in the slot correspondence table 401 while the identification number of a cradle is displayed on the screen 504. To achieve different ways of using them as above, a fixed correspondence relation between a port address and a cradle ID is pre-stored in the hard disk 307. Then, the correspondence relation is read out to, for example, the RAM 303 in step S107. The operator is enabled to confirm that the receivers that were equipped on the patients with the patient IDs "02" and "01" are respectively mounted on the cradles 101 and 102, by merely looking at the screen 504.

Further, the screen 504 shown in Fig. 6 also displays a button with "start transfer" written on it. The description now returns to Fig. 4. The operator uses, for example, a mouse to click on the button in order to instruct the observation apparatus 301 to start the transfer of image data, and thereby the process proceeds from step S107 to step S108. The image data stored in the two receivers is transferred (i.e., transmitted) to the observation apparatus 301 in step S108.

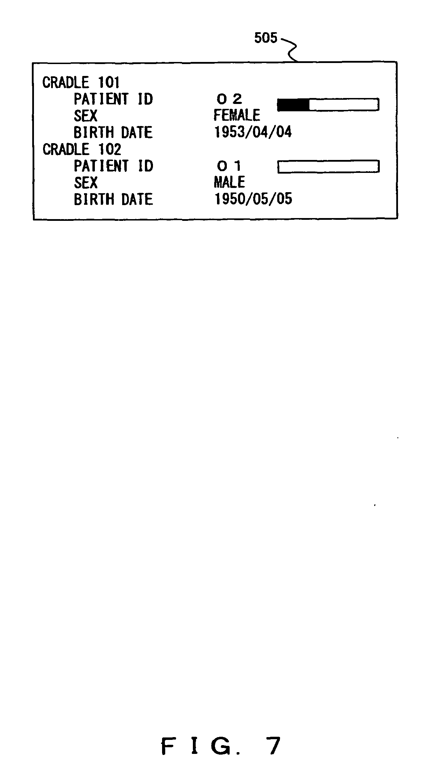

The first embodiment assumes that the sequence of transfer is predetermined as "the cradle 101 first and the cradle 102 second". Therefore, step S108 starts with the transfer of the image data to the observation apparatus 301 from the receiver 202 mounted on the cradle 101. Then, after the completion of the transfer from the receiver 202, the transfer of the image data to the observation apparatus 301 from the receiver 201 mounted on the cradle 102 is carried out. The transfer of the image data in step S108 is controlled by the CPU 304 of the observation apparatus 301. That is, the control circuit 207 of the receiver 201 and the control circuit 208 of the receiver 202 respectively transfer the image data to the observation apparatus 301 at timings designated by the observation apparatus 301. During the transfer of the image data, a screen 505 as shown in Fig. 7 is displayed on the LCD 306 of the observation apparatus 301.

In comparison with Fig. 6, Fig. 7 differs from it in that there is no "start transfer" button, but instead progress bars indicating the progress of the transfer of image data are displayed corresponding to both cradle IDs. The screen 505 shown in Fig. 7 exemplifies the stage in which approximately one-third of the entirety of the transfer of the image data from the cradle 101 to the observation apparatus 301 is completed, and the transfer of the image data from the cradle 102 to the observation apparatus 301 is not yet started.

As described above, the first embodiment is configured such that merely clicking on the "start transfer" button causes sequential transfers of image data from the two receivers to the observation apparatus 301. Therefore, the operator can be engaged in other work without any interruptions until the transfer from both of the receivers 202 and 201 is completed after clicking on the "start transfer" button. Further, it is obvious that three or more receivers may be used. Therefore, the work efficiency of the operator in the first embodiment of the present invention can be better than those in conventional arts, even when a receiver having a built-in patient information storage unit is used.

In the meantime, when a plurality of patients are examined one after another, there is sometimes a need to equip new patients with receivers one after another by reusing the receivers from which the transfer of image data is completed. Even in such a case, by merely taking a glance at the screen 505 of Fig. 7, the operator can recognize whether or not there is a progress bar that indicates a 100% progress state, that is, whether or not there is a receiver that is ready for a reuse since the transfer of image data was completed therefrom. Further, the operator can easily find out which receiver accumulates which patient's image data and can find out the progress of the transfer of image data from each receiver. Therefore, it is also easy to devise a plan for reusing the receivers in accordance with the progress.

Meanwhile, the CPU 304 of the observation apparatus 301 may also control the LCD 306 regarding content other than the progress bars of the screen 505 such that display contents are changed in accordance with the transfer state of image data; for example, the state of "transfer in progress" and the state of "transfer complete".

As an example, the CPU 304 may control colors, brightness, blinking patterns, and the like, which are used when information corresponding to a receiver (i.e., the cradle ID and the patient information) is displayed on the screen 505. Alternatively, the CPU 304 may control the LCD 306 such that it displays the information while changing the combination of colors, brightness, blinking patterns, and the like in accordance with the transfer state of image data. It is possible to clearly show a receiver currently transferring image data on the screen 505 by means of such a control in accordance with the transfer state of image data.

Further, if there is a receiver ready to be reused, the operator can also recognize on which cradle the receiver is mounted by looking only at the screen 505.

This is because progress bars are displayed associated with each cradle ID, as shown in Fig. 7. The operator can detach a receiver that is ready to be reused from the cradle and use it for examining a new patient. Here, the operator preferably compares the patient information displayed on the LCD of the receiver with the screen 505 of the LCD 306 of the observation apparatus 301 in order to prevent erroneously detaching, from the cradle, a receiver from which the transfer of image data has not yet been completed.

As an example, when the data transfer from the receiver 202 mounted on the cradle 101 further progresses and is completed in the example shown in Fig. 7, the operator looks at the progress bar displayed corresponding to the cradle 101 and accordingly recognizes that it shows a 100% progress. That is, the operator recognizes that the receiver can be detached from the cradle 101.

In this event, the operator compares the screen 505 on the LCD 306 of the observation apparatus 301 with the screen on the LCD 204 of the receiver 202 mounted on the cradle 101 and confirms that the same patient information is displayed. In this way, the receiver 201, which has not completed the transfer of image data, is prevented from being wrongly detached from the cradle 102. Further, if a label on which the cradle ID is written is pre-attached to each cradle as described above, it is possible to further securely prevent a mistake by the operator further comparing the cradle ID written on the label with the cradle ID displayed in the screen 505.

As described above, the first embodiment makes it possible to visually recognize from its appearance a receiver which completed transferring image data. Therefore, by carrying out a simple operation the receiver can be reused efficiently while preventing a mistake. Further, in comparison with the case of using a receiver configured to accumulate image data in a removable storage medium, since an operator does not need to perform the operation for attaching or detaching a storage medium, the first embodiment of the present invention requires only a light workload of the operator and is excellent in operability.

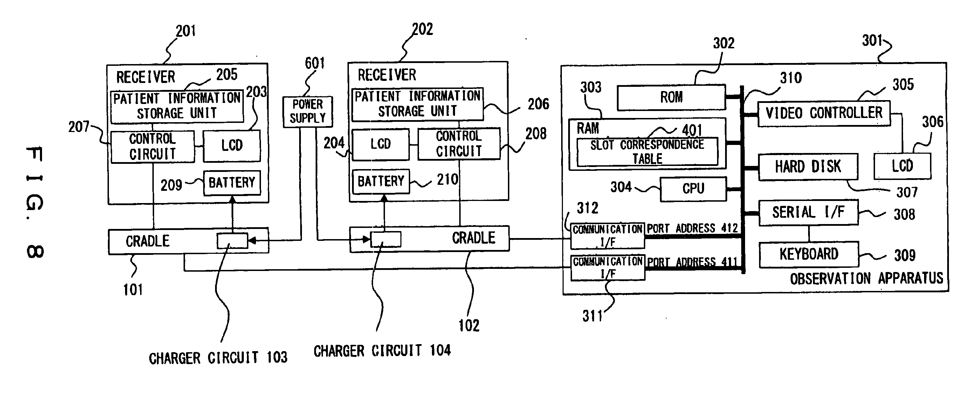

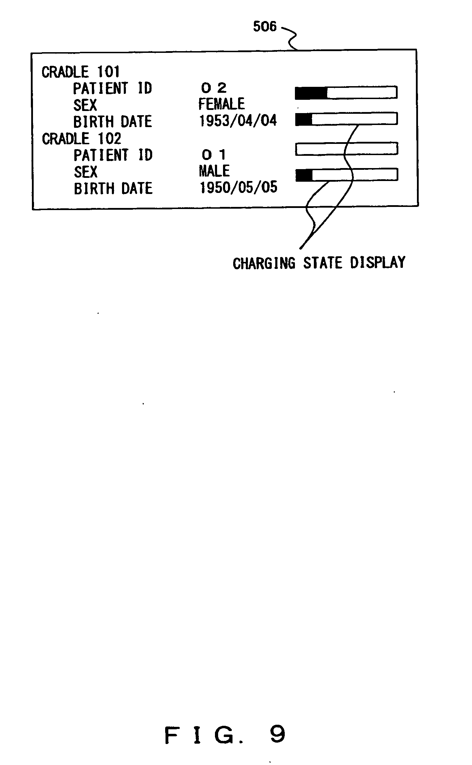

Next is a description of a second embodiment of the present invention by referring to Figs. 8 and 9. Note that the same reference signs as in the first embodiment are assigned to constituent components common to those of the first embodiment, and the description is omitted as appropriate.

Fig. 8 is a block diagram showing the configuration of a capsule-type endoscope system according to the second embodiment of the present invention. The difference from the first embodiment shown in Fig. 2 lies in following (a) and (b).
(a) The cradles 101 and 102 respectively include charger circuits 103 and 104, which are connected to an external power supply 601; and
(b) The receivers 201 and 202 respectively include batteries 209 and 210 that are charged respectively by way of the charger circuits 103 and 104.

That is, the battery 209 is a rechargeable battery. When the receiver 201 is mounted on the cradle 101 or 102, the battery 209 is electrically connected to the charger circuit 103 or 104 so as to be charged from the power supply 601 by way of the charger circuit 103 or 104. This is similar for the battery 210.

Fig. 9 is a diagram exemplifying the screen 506 on the LCD 306 of the observation apparatus 301 displayed when image data is transferred from a receiver to the observation apparatus 301 in the second embodiment. The difference between the screen 505 in step S108 of the first embodiment shown in Fig. 7 and the screen 506 in Fig. 9 is that the progress of the charging associated with each cradle ID is also displayed in Fig. 9. The batteries 209 and 210 are simultaneously and parallelly charged on the two cradles as shown in Fig. 9.

As such, the second embodiment is configured to display the progress of charging and that of an image data transfer together on one screen 506. Therefore, the operator can easily identify and reuse a receiver that is ready to be reused after completing a data transfer and sufficient charging. Further, the time can be effectively used by charging the battery in parallel with the image data transfer.

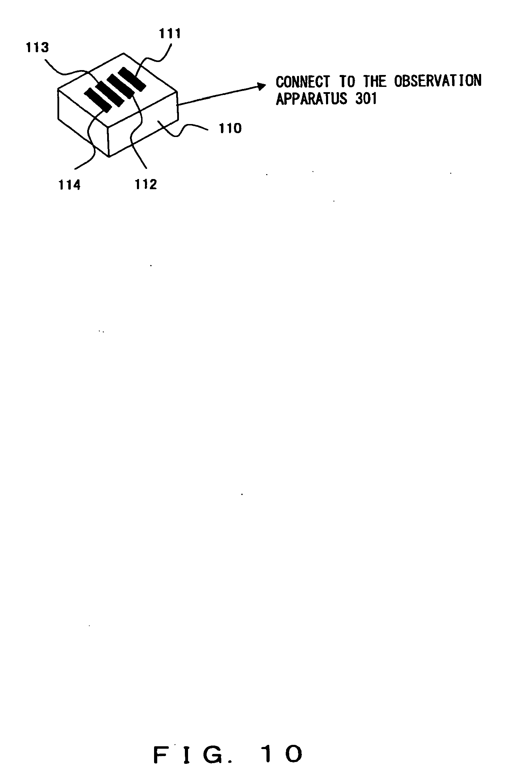

Next is a description of a third embodiment of the present invention by referring to Figs. 10, 11 and 12. Note that the same reference signs are assigned to constituent components common to those of the first and second embodiments, and the description is omitted as appropriate.

Fig. 10 is a perspective view of a cradle used in the third embodiment of the present invention. In the third embodiment, a cradle has a plurality of slots for inserting receivers. As an example, the cradle 110 in Fig. 10 has four slots 111 through 114. In the third embodiment, mounting units are embodied by the slots 111 through 114 of the cradle, as with the case of the first and second embodiments. A plurality of receivers can be collectively connected to an observation apparatus 301 by means of the cradle 110 having a plurality of slots corresponding to the mounting units and via a cable connecting the cradle 110 to the observation apparatus 301.

If there are many receivers to be simultaneously connected to the observation apparatus 301 in order to transfer pieces of image data en masse, there is the advantage that the cradle 110 occupies a smaller space than in the situation in which many cradles like those in the first embodiment are used.

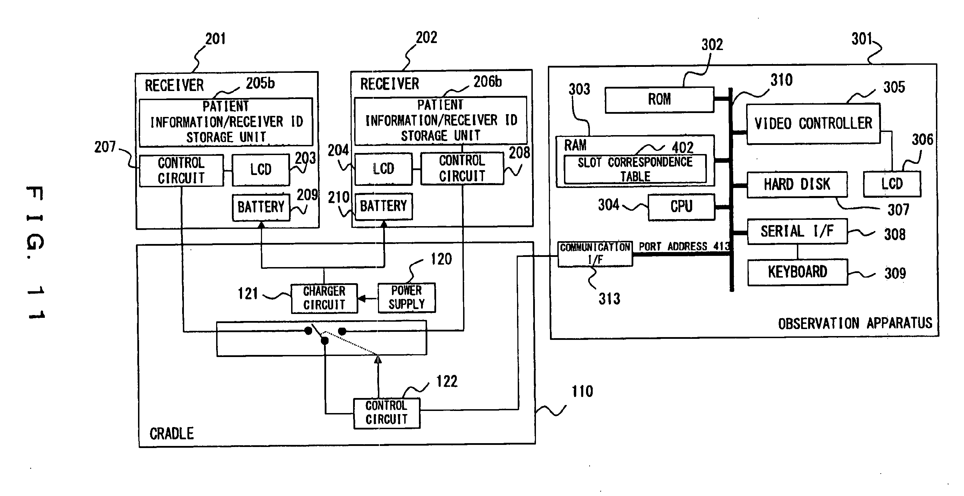

Fig. 11 is a block diagram showing the configuration of a capsule-type endoscope system according to the third embodiment of the present invention. The differences from the second embodiment shown in Fig. 8 are those resulted from the cradle 110 having a plurality of slots. Note that Fig. 11 indicates only a part corresponding to two of the four slots 111 through 114 due to space limitations.

In Fig. 11, only one cradle 110 is connected to the observation apparatus 301, and therefore it is sufficient for the observation apparatus 301 to have only one communication I/F 313. A port address 413 is assigned to the communication I/F 313.

The cradle 110 includes a control circuit 122 for selecting a slot and controlling the transmission of data between a receiver inserted into the selected slot and the observation apparatus 301. As with step S108 of the first embodiment, the third embodiment is also made such that the control circuit 122 changes over the selections of slots at a timing that depends on an instruction from the CPU 304 of the observation apparatus 301. Since the changeover is carried out automatically, the degree of operator intervention is reduced in comparison with the conventional system.

As with the cradles 101 and 102 of Fig. 8, the cradle 110 also includes a charger circuit 121. The charger circuit 121 is configured to charge a plurality of receivers inserted in a plurality of slots simultaneously and parallelly. The power supply 120 supplies the charger circuit 121 with the power.

The configurations of receivers 201 and 202 shown in Fig. 11 are approximately the same as those of the receivers 201 and 202 shown in Fig. 8, with an exception that patient information/receiver ID storage units 205b and 206b are equipped in place of the patient information storage units 205 and 206 shown in Fig. 8. The patient information/receiver ID storage units 205b and 206b also store a receiver ID identifying a receiver, in addition to patient information and image data, which are stored in the patient information storage units 205 and 206. A receiver ID may be, for example, a production serial number which is assigned when a receiver is produced and which is stored in the patient information/receiver ID storage unit 205b or 206b.

Assume that in the following description, the receiver IDs of the receivers 201 and 202 are respectively "3457" and "3458". That is, the patient information/receiver ID storage units 205b and 206b respectively pre-store "3457" and "3458" as the receiver IDs.

Further assume that, as with the first embodiment, the receivers 201 and 202 are respectively initialized with the patient information related to the patients whose patient IDs are "01" and "02", respectively, and are used for examining the respective patients. That is, the patient information including the patient ID "01" is written to the patient information/receiver ID storage unit 205b of the receiver 201 as a result of the initialization. The patient information including the patient ID "02" is likewise written to the patient information/receiver ID storage unit 206b of the receiver 202 as a result of the initialization.

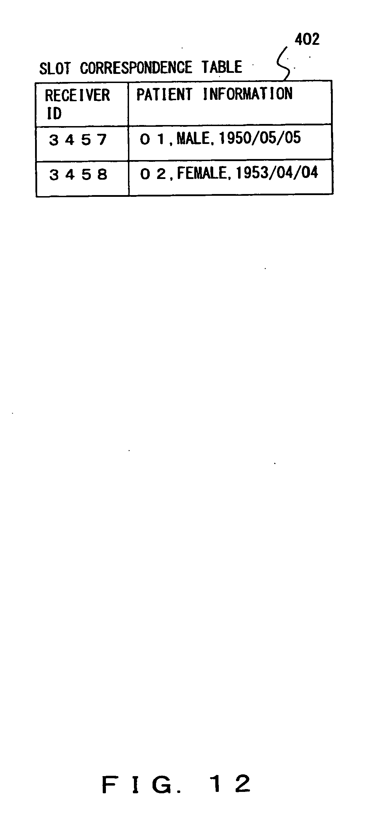

Fig. 12 is a diagram exemplifying a slot correspondence table according to the third embodiment of the present invention. Compared with the slot correspondence table 401 of the first embodiment shown in Fig. 3, the difference is that a receiver ID is used in place of the port address. Fig. 12 specifically shows an example of the patient information being constituted by patient ID, sex and birth date.

As with the case of the first embodiment described by referring to steps S105 and S106 shown in Fig. 4, a record is created in the slot correspondence table 402 of Fig. 12, triggered by the insertion of a receiver, after completing an examination, into either slot of the cradle 110.

When, for example, the receiver 201 is inserted into the slot 112, the insertion is detected by the communication I/F 313 by way of the cradle 110 and is reported to the CPU 304. Using this report as a trigger, the CPU 304 instructs the receiver 201 to transmit the receiver ID and the patient information stored in the patient information/receiver ID storage unit 205b. As a result, receiver ID "3457" and the patient information constituted by patient ID "01", sex "male", and birth date "1950/05/05" are read from the patient information/receiver ID storage unit 205b and transmitted to the observation apparatus 301. Then, the first record in the slot correspondence table 402 is created. The second record is also created in a similar manner.

A receiver ID may be, for example, the production serial number of a receiver, or any other number. The third embodiment is configured to pre-attach a label on which a receiver ID is written to each receiver. When image data is transmitted from a receiver to the observation apparatus 301, the CPU 304 controls, by way of the video controller 305, the LCD 306 such that it displays a screen in which receiver IDs are substituted for the cradle IDs in the screen 506 shown in Fig. 9. This enables the operator to simply recognize the progress of an image data transmission from each receiver and the progress of charging by comparing the labels and the LCDs 203 and 204 of the receivers 201 and 202 with the LCD 306 of the observation apparatus 301. Further, since both a receiver ID and patient information are visually recognizable, the operator can more securely select a receiver which has completed an image data transmission and charging than in the case of using only either a receiver ID or patient information.

Note that the present invention may be embodied with various modifications, in lieu of being limited to the embodiments described above. The following provides some examples.

It is clearly possible for the operator to mount the receiver 201 onto the cradle 101 and the receiver 202 onto the cradle 102 in step S105 shown in Fig. 4. Also in this case, records in the slot correspondence table are created in a similar manner to the above description.

The sequence of an image data transfer from a plurality of receivers to the observation apparatus 301 arbitrarily changes in accordance with the embodiments. Although the first embodiment described above is configured to predetermine the sequence, it is also possible that the transfer is made in the sequence designated by an operator via the keyboard 309 or the like. Alternatively, when image data are transferred from, for example, two receivers 201 and 202, the CPU 304 may control and change over the transfer from the receiver 201 and that from the receiver 202 so that a constant amount of image data is transferred alternately from the receivers 201 and 202 to the observation apparatus 301.

The observation apparatus 301 may include a plurality of CPUs. The CPUs may be configured such that they can respectively and independently control a plurality of communication I/Fs. In such a case, each of the plurality of CPUs may independently control the transfer so as to carry out the transfers from a plurality of receivers simultaneously and parallelly via a plurality of cradles connected to respective communication I/Fs.

Although a plurality of cradles are separately connected to the observation apparatus 301 in Figs. 2 and 8, it is also possible to adopt a configuration in which one hub device is connected to the observation apparatus 301; and a plurality of cradles are connected to the hub device, respectively. Further, a cradle having only one slot and a cradle having a plurality of slots may be intermixed. A cradle may be connected to the observation apparatus 301 by way of a wireless communication, in place of a cable.

Further, as described above, there are various types of cradles depending on the embodiments. It varies with the type, for example, whether or not a cradle includes a charger circuit, or how many slots one cradle has. There may be, however, an embodiment in which a plurality of cradles of different types are intermixed and used. In the case where different types of cradles are intermixed, the above described plurality of mounting units are implemented by a plurality of slots distributed to a plurality of cradles.

As is clear from the comparison between the slot correspondence table 401 shown in Fig. 3 and the slot correspondence table 402 shown in Fig. 12, the present invention can be implemented as long as the observation apparatus 301 is capable of managing the correspondence relation between a receiver and a patient ID. That is, as long as information identifying a receiver directly or indirectly is associated with information identifying a patient, the correspondence relation may be managed in a form other than a table form such as the slot correspondence table.

An example of information directly identifying a receiver is the receiver ID noted in the third embodiment. The following (a) through (c) are the examples of information indirectly identifying a receiver.
(a) The ID of the slot in which a receiver is inserted, for the case in which a cradle having a plurality of slots is used.
(b) The ID of a cradle, for the case in which a slot and a cradle correspond to each other in a one-to-one manner.
(c) The port address of a communication I/F, for the case in which a slot, a cradle, and the communication I/F of the observation apparatus 301 correspond to each other in a one-to-one-to-one manner.

Further, as an example, if a slot and a cradle correspond to each other in a one-to-one manner, the production serial number of a cradle may be used as the information indirectly identifying a receiver. In this case, if a label on which the production serial number is written is attached to the surface of a cradle, the operator can visually recognize the production serial number of the cradle easily.

Further, as described above, in the first embodiment, a port address is used in the slot correspondence table 401 and while a cradle ID is displayed on the LCD 306 of the observation apparatus 301. Accordingly, a fixed correspondence relation between the port address and the cradle ID is managed. It is clear that such a management may be unnecessary depending on the content to be displayed on the LCD 306 and that the information to be managed may be different depending on the embodiments.

A receiver may further be equipped with an illumination device, such as a lamp, as a measure to prevent a receiver that has not completed an image data transfer from being mistakenly detached when a receiver is to be detached from a cradle to reuse it. Whether or not a data transfer has been completed is indicated by means of the state of the lighting or the blinking of a lamp, the color of the lamp, or the combination of these, such that it is more certain that the mistake is prevented by the operator checking the lamp in addition to the LCD.

Further, a cradle may be equipped with a locking mechanism for mechanically locking a receiver in a slot of the cradle in order to prevent a receiver that has not completed an image data transfer from being wrongly detached from the cradle. That is, such a locking mechanism locks up and fixedly retains a receiver when an image data transfer is started so as to prevent the receiver from being detached from the slot of the cradle and unlocks when the image data transfer is completed.

Alternatively, the CPU 304 of the observation apparatus 301 may instruct the control circuit of a receiver which has completed an image data transfer to delete the entirety of data. For example, when an image data transfer from the receiver 201 is completed, the control circuit 207 of the receiver 201 deletes the entirety of the data stored in the patient information storage unit 205 in accordance with the instruction from the CPU 304 of the observation apparatus 301.

As a result, data to be displayed on the display unit 203 realizing the first display unit disappears from the receiver 201. The control circuit 207 may control the display unit 203 such that it displays nothing after deleting the data. This enables the operator to easily recognize that the only receiver that may be detached from the cradle is the receiver displaying nothing on the display unit. Therefore, such a data deletion and a display control also make it possible to prevent a receiver which has not completed an image data transfer from being wrongly detached from the cradle.

Further, the screen 505 of Fig. 7 and the screen 506 of Fig. 9 exemplify a progress bar as the display of progress; it is also possible to indicate the progress by means of characters such as "will complete in n minutes". Further, the CPU 304 of the observation apparatus 301 may control the LCD 306 such that it displays the progress of transferring image data by displaying on the screens 505 or 506 the speed of the image data transfer in units of, for example, bps (bits per second). Furthermore, the progress of an image data transfer and/or that of charging may be displayed on a receiver.

Note that pieces of the patient information displayed on the LCDs of the receiver and the observation apparatus 301 are not limited to being the exemplified combination. Depending on the embodiments, another piece of information, such as a name, may be further displayed or a portion of the exemplified patient information may not be displayed.

As described above, according to the present invention, it is possible to together connect to the observation apparatus a plurality of receivers equipped with a built-in patient information storage unit, so as to transfer image data en masse. It is therefore possible to reduce the time an operator is detained or the number of times the operator has to interrupt his/her other work. Further, the operator can easily discern a receiver that is ready for reuse after the completion of an image data transfer by comparing the LCDs of the receiver and the observation apparatus. Therefore, a receiver can be reused efficiently without degrading operability.

## Claims

1. A capsule-type endoscope system, including: a plurality of receivers for receiving, from a capsule-type endoscope by way of a wireless communication, image data of an observation image of an inside of a body of an examinee imaged by the capsule-type endoscope; a plurality of mounting units for respectively mounting the plurality of receivers; and an information processing apparatus connected to the plurality of receivers by way of the mounting units, wherein
each of the plurality of receivers includes a first storage unit and a first display unit,
the first storage unit stores examinee identification information identifying the examinee and the received image data,
the first display unit displays the examinee identification information or examinee information about the examinee who is associated with the examinee identification information,
the information processing apparatus includes:
a management unit for receiving the examinee identification information from each of the plurality of receivers by way of the mounting unit and for managing a correspondence relation between receiver identification information identifying the receiver or mounting unit identification information identifying the mounting unit on which the receiver is mounted and the received examinee identification information;
a second storage unit; and
a second display unit,
the image data is transmitted to the information processing apparatus by way of the mounting unit and is stored in the second storage unit,
the second display unit displays the correspondence relation by displaying at least a part of the examinee identification information displayed on the first display unit or at least a part of the examinee information displayed on the first display unit, and
the second display unit further displays a progress of the transmission of the image data corresponding to each of the plurality of receivers.

2. The capsule-type endoscope system according to claim 1, wherein the plurality of mounting units are a plurality of slots provided at at least one cradle device.

3. The capsule-type endoscope system according to claim 1, wherein the plurality of mounting units are slots provided at each of a plurality of cradle devices.

4. The capsule-type endoscope system according to claim 3, wherein the mounting unit identification information is either port identification information identifying a plurality of ports which are comprised in the information processing apparatus and to which the plurality of cradle devices are respectively connectable, or cradle device identification information identifying the plurality of cradle devices.

5. A program for a capsule-type endoscope system which includes: a plurality of receivers for receiving, from a capsule-type endoscope by means of a wireless communication, image data of an observation image of an inside of a body of an examinee imaged by the capsule-type endoscope, so as to store the image data, and for storing examinee identification information that identifies the examinee; a plurality of mounting units enabling the plurality of receivers to be respectively mounted; and an information processing apparatus connected to the plurality of receivers by way of the mounting units, wherein the program directs the information processing apparatus to execute:
a step of detecting that any of the receivers is mounted on any of the mounting unit;
a step of receiving the examinee identification information from the detected receiver by way of the mounting unit;
a step of associating receiver identification information identifying the detected receiver or mounting unit identification information identifying the detected mounting unit with the received examinee identification information, so as to store a correspondence relation therebetween;
a step of displaying the correspondence relation by displaying at least a part of the examinee identification information displayed on each of the plurality of receivers or at least a part of examinee information that is about an examinee who is associated with the examinee identification information and that is displayed on each of the plurality of receivers; and
a step of receiving the image data transmitted from the detected receiver by way of the mounting unit while displaying at least either a progress of the transmission or a transfer speed of the transmission.

6. The capsule-type endoscope system according to claim 3, wherein at least one of the plurality of cradle devices includes a charger circuit for charging the receiver mounted on the slot while the receiver transmits the image data.

7. The capsule-type endoscope system according to claim 1, wherein at least either the first display unit of the receiver or the second display unit of the information processing apparatus displays a charging state of the receiver.

8. The capsule-type endoscope system according to claim 1, wherein the receiver includes an illumination device for indicating a transfer state of the image data.

9. The capsule-type endoscope system according to claim 1, wherein at least either the first display unit of the receiver or the second display unit of the information processing apparatus displays at least either a figure or a character indicating a transfer state of the image data.

10. The program according to claim 5, wherein the program further directs the information processing device to execute
at least either a step of indicating a charging state of the receiver or a step of controlling the receiver to indicate the charging state of the receiver.

11. The program according to claim 5, wherein the program further directs the information processing device to execute
at least either a step of indicating a transfer state of the image data by means of at least either a figure or a character or a step of controlling the receiver to indicate the transfer state of the image data by means of at least either a figure or a character.

12. The capsule-type endoscope system according to claim 1, comprising a control unit for controlling the second display unit to display information corresponding to the receiver while changing at least one of a color, brightness, and a blinking pattern in accordance with the progress of the transmission of the image data, when the image data stored in the first storage unit of the plurality of receivers is transmitted to the information processing apparatus.

13. The capsule-type endoscope system according to claim 1, comprising a control unit for selecting a sequence of transferring the image data stored in the first storage unit of each of the plurality of receivers to the information processing apparatus.

14. The capsule-type endoscope system according to claim 1, comprising
a control unit for selecting one of a plurality of choices of a transfer method for transferring each piece of the image data stored in the first storage unit of each of the plurality of receivers to the information processing apparatus, wherein
the plurality of choices include at least one of
a serial transfer that carries out, sequentially for each of the plurality of receivers, a process for transferring an entirety of the image data from the first storage unit of one of the receivers to the information processing apparatus,
an alternate transfer that repeats transferring each part of the image data stored in each of the first storage unit alternately from the plurality of receivers to the information processing apparatus, and
a parallel transfer that simultaneously and parallelly transfers each piece of the image data from the first storage unit of each of the plurality of receivers to the information processing apparatus.

15. The capsule-type endoscope system according to claim 1, comprising a retention unit for mechanically fixing the receiver transferring the image data in order to prevent the receiver from being detached from the mounting unit.

16. The capsule-type endoscope system according to claim 1, wherein at least either the first display unit of the receiver or the second display unit of the information processing apparatus indicates at least either a transfer state of the image data or a transfer speed of the image data by means of at least either a figure or a character.

17. The program according to claim 5, wherein the program further directs the information processing apparatus to execute
a step of displaying information corresponding to the receiver while changing at least one of a color, brightness, and a blinking pattern in accordance with the progress of the transmission of the image data, when the image data stored in the plurality of receivers is transmitted to the information processing apparatus.

18. The program according to claim 5, wherein the program further directs the information processing apparatus to execute
a step of selecting a sequence of transferring the image data stored in the plurality of receivers to the information processing apparatus.

19. The program according to claim 5, wherein the program further directs the information processing apparatus to execute
at least either a step of indicating at least either a transfer state of the image data or a transfer speed of the image data by means of at least either a figure or a character, or a step of controlling the receiver to indicate at least either the transfer state of the image data or the transfer speed of the image data by means of at least either a figure or a character.

20. A method to be executed by a capsule-type endoscope system which includes: a plurality of receivers; a plurality of mounting units enabling the plurality of receivers to be respectively mounted; and the information processing apparatus connected to the plurality of receivers by way of the mounting units, wherein the information processing apparatus
detects that any of the receivers is mounted on any of the mounting units,
receives, from the detected receiver by way of the mounting unit, examinee identification information identifying an examinee who is an observation target of image
data of an observation image of an inside of a body and whose body has been imaged from inside by a capsule-type endoscope, the image data having been received from the capsule-type endoscope by way of a wireless communication and being stored in the detected receiver,
associates receiver identification information identifying the detected receiver or mounting unit identification information identifying the detected mounting unit with the received examinee identification information, so as to store a correspondence relation therebetween,
displays the correspondence relation by displaying at least a part of the examinee identification information displayed on each of the plurality of receivers or at least a part of examinee information that is about an examinee who is associated with the examinee identification information and that is displayed on each of the plurality of receivers,
receives the image data transmitted from the detected receiver by way of the mounting unit, and
displays at least either a progress of the transmission or a transfer speed of the transmission.

## Patentansprüche

1. Kapselartiges Endoskopsystem mit:
einer Vielzahl von Empfängern zum Empfangen von Bilddaten eines Beobachtungsbilds vom Inneren eines Patientenkörpers, das durch ein kapselartiges Endoskop abgebildet wird, von dem kapselartigen Endoskop über eine drahtlose Kommunikation; einer Vielzahl von Anbringeinheiten zum jeweiligen Anbringen der Vielzahl von Empfängern; und einer Informationsverarbeitungsvorrichtung, die über die Anbringeinheiten mit der Vielzahl von Empfängern verbunden ist, wobei
jeder der Vielzahl von Empfängern eine erste Speichereinheit und eine erste Anzeigeeinheit aufweist,
die erste Speichereinheit Patientenidentifikationsinformationen speichert, die den Patienten und die empfangenen Bilddaten identifiziert,
die erste Anzeigeeinheit die Patientenidentifikationsinformationen oder Patienteninformationen über den zu den Patientenidentifikationsinformationen gehörenden Patienten anzeigt,
die Informationsverarbeitungsvorrichtung aufweist:
eine Verwaltungseinheit zum Empfangen der
Patientenidentifikationsinformationen von jedem der Vielzahl von Empfängern über die Anbringeinheit und zum Verwalten einer Übereinstimmungsbeziehung zwischen Empfängeridentifikationsinformationen, welche den Empfänger identifizieren, oder Informationen zur Anbringeinheit-Identifikation, welche die Anbringeinheit identifizieren, an welcher der Empfänger angebracht ist, und
den empfangenen Patientenidentifikationsinformationen;
eine zweite Speichereinheit; und
eine zweite Anzeigeeinheit,
die Bilddaten über die Anbringeinheit zur
Informationsverarbeitungsvorrichtung übertragen und in der zweiten Speichereinheit gespeichert werden,
die zweite Anzeigeeinheit die Übereinstimmungsbeziehung anzeigt, indem sie mindestens einen Teil der Patientenidentifikationsinformationen, die auf der ersten Anzeigeeinheit angezeigt werden, oder mindestens einen Teil der Patienteninformationen anzeigt, die auf der ersten Anzeigeeinheit angezeigt werden,
und
die zweite Anzeigeeinheit des Weiteren einen Fortschritt der Übertragung der Bilddaten anzeigt, die jedem der Vielzahl von Empfängern entsprechen.

2. Kapselartiges Endoskopsystem nach Anspruch 1, wobei die Vielzahl von Anbringeinheiten eine Vielzahl von Steckplätzen ist, die an mindestens einer Dockingstation vorgesehen sind.

3. Kapselartiges Endoskopsystem nach Anspruch 1, wobei die Vielzahl von Anbringeinheiten Steckplätze sind, die an jeder einer Vielzahl von Dockingstationen vorgesehen sind.

4. Kapselartiges Endoskopsystem nach Anspruch 3, wobei die Informationen zur Anbringeinheit-Identifikation entweder Informationen zur Port-Identifikation, die eine Vielzahl von Ports identifizieren, die in der Informationsverarbeitungsvorrichtung enthalten sind und an welche die Vielzahl von Dockingstationen jeweils anschließbar sind, oder Informationen zur Dockingstation-Identifikation sind, welche die Vielzahl von Dockingstationen identifizieren.

5. Programm für ein kapselartiges Endoskopsystem, das aufweist:
eine Vielzahl von Empfängern zum Empfangen von Bilddaten eines Beobachtungsbilds vom Inneren eines Patientenkörpers, das durch ein kapselartiges Endoskop abgebildet wird, von dem kapselartigen Endoskop über eine drahtlose Kommunikation, um die Bilddaten zu speichern, und zum Speichern von Patientenidentifikationsinformationen, die den Patienten identifizieren; eine Vielzahl von Anbringeinheiten zum jeweiligen Anbringen der Vielzahl von Empfängern; und eine Informationsverarbeitungsvorrichtung, die über die Anbringeinheiten mit der Vielzahl von Empfängern verbunden ist, wobei das Programm die Informationsverarbeitungsvorrichtung anweist, auszuführen:
einen Schritt zum Ermitteln, dass einer der Empfänger an einer der Anbringeinheiten angebracht ist;
einen Schritt zum Empfangen der Patientenidentifikationsinformationen vom ermittelten Empfänger über die Anbringeinheit;
einen Schritt zum Zuordnen von Empfängeridentifikationsinformationen, die den ermittelten Empfänger identifizieren, oder von Informationen zur Anbringeinheit-Identifikation, welche die ermittelte Anbringeinheit identifizieren, zu den empfangenen Patientenidentifikationsinformationen, um eine Übereinstimmungsbeziehung zwischen ihnen zu speichern;
einen Schritt zum Anzeigen der Übereinstimmungsbeziehung durch Anzeigen zumindest eines Teils der Patientenidentifikationsinformationen, die auf jedem der Vielzahl von Empfängern angezeigt werden, oder zumindest eines Teils der Patienteninformationen, die einen Patienten betreffen, der den Patientenidentifikationsinformationen zugeordnet ist und die auf jedem der Vielzahl von Empfängern angezeigt werden; und
einen Schritt zum Empfangen der Bilddaten, die von dem ermittelten Empfänger über die Anbringeinheit übertragen werden, während ein Fortschritt der Übertragung und/oder eine Übertragungsrate der Übertragung angezeigt wird.

6. Kapselartiges Endoskopsystem nach Anspruch 3, wobei mindestens eine der Vielzahl von Dockingstationen eine Ladeschaltung zum Aufladen des an dem Steckplatz angebrachten Empfängers aufweist, während der Empfänger die Bilddaten überträgt.

7. Kapselartiges Endoskopsystem nach Anspruch 1, wobei die erste Anzeigeeinheit des Empfängers und/oder die zweite Anzeigeeinheit der Informationsverarbeitungsvorrichtung einen Ladezustand des Empfängers anzeigt.

8. Kapselartiges Endoskopsystem nach Anspruch 1, wobei der Empfänger eine Beleuchtungsvorrichtung zum Angeben eines Übertragungszustands der Bilddaten aufweist.

9. Kapselartiges Endoskopsystem nach Anspruch 1, wobei die erste Anzeigeeinheit des Empfängers und/oder die zweite Anzeigeeinheit der Informationsverarbeitungsvorrichtung eine Ziffer und/oder einen Buchstaben anzeigt, die/der einen Übertragungszustand der Bilddaten angibt/angeben.

10. Programm nach Anspruch 5, wobei das Programm des Weiteren die Informationsverarbeitungsvorrichtung anweist, auszuführen:
einen Schritt zum Angeben eines Ladezustandes des Empfängers und/oder einen Schritt zum Steuern des Empfängers, um den Ladezustand des Empfängers anzugeben.

11. Programm nach Anspruch 5, wobei das Programm des Weiteren die Informationsverarbeitungsvorrichtung anweist, auszuführen:
einen Schritt zum Angeben eines Übertragungszustandes der Bilddaten mittels einer Ziffer und/oder eines Buchstabens und/oder einen Schritt zum Steuern des Empfängers, um den Übertragungszustand der Bilddaten mittels einer Ziffer und/oder eines Buchstabens anzugeben.

12. Kapselartiges Endoskopsystem nach Anspruch 1, das eine Steuereinheit zum Steuern der zweiten Anzeigeeinheit aufweist, um den Empfänger betreffende Informationen anzuzeigen, während sich zumindest ein Parameter von Farbe, Helligkeit und Blinkmuster gemäß dem Fortschritt der Übertragung der Bilddaten ändert, wenn die Bilddaten, die in der ersten Speichereinheit der Vielzahl von Empfängern gespeichert sind, zur Informationsverarbeitungsvorrichtung übertragen werden.

13. Kapselartiges Endoskopsystem nach Anspruch 1, das eine Steuereinheit zum Auswählen einer Abfolge des Übertragens der Bilddaten, die in der ersten Speichereinheit von jedem der Vielzahl von Empfängern gespeichert sind, zur Informationsverarbeitungsvorrichtung aufweist.

14. Kapselartiges Endoskopsystem nach Anspruch 1, das aufweist
eine Steuereinheit zum Auswählen einer aus einer Vielzahl von Wahlmöglichkeiten eines Übertragungsverfahrens zum Übertragen jedes Teils der Bilddaten, die in der ersten Speichereinheit von jedem der Vielzahl von Empfängern gespeichert sind, zur Informationsverarbeitungsvorrichtung, wobei
die Vielzahl von Wahlmöglichkeiten zumindest eine der folgenden aufweist
eine serielle Übertragung, die für jeden der Vielzahl von Empfängern ein Verfahren zum Übertragen einer Gesamtheit der Bilddaten aus der ersten Speichereinheit eines der Empfänger zur Informationsverarbeitungsvorrichtung sequenziell ausführt,
eine alternierende Übertragung, die das Übertragen jedes Teils der Bilddaten, die in jeder der ersten Speichereinheiten gespeichert sind, abwechselnd von der Vielzahl von Empfängern zur Informationsverarbeitungsvorrichtung wiederholt, und
eine parallele Übertragung, die jeden Teil der Bilddaten von der ersten Speichereinheit jedes der Vielzahl von Empfängern zur Informationsverarbeitungsvorrichtung gleichzeitig und parallel überträgt.

15. Kapselartiges Endoskopsystem nach Anspruch 1, das eine Halteeinheit zum mechanischen Fixieren des Empfängers, der die Bilddaten überträgt, aufweist, um ein Abnehmen des Empfängers aus der Anbringeinheit zu verhindern.

16. Kapselartiges Endoskopsystem nach Anspruch 1, wobei die erste Anzeigeeinheit des Empfängers und/oder die zweite Anzeigeeinheit der Informationsverarbeitungsvorrichtung einen Übertragungszustand der Bilddaten und/oder eine Übertragungsrate der Bilddaten mittels einer Ziffer und/oder eines Buchstabens angibt.

17. Programm nach Anspruch 5, wobei das Programm des Weiteren die Informationsverarbeitungsvorrichtung anweist, auszuführen:
einen Schritt zum Anzeigen von dem Empfänger entsprechenden Informationen, während sich zumindest ein Parameter von Farbe, Helligkeit und Blinkmuster gemäß dem Fortschritt der Übertragung der Bilddaten ändert, wenn die in der Vielzahl von Empfängern gespeicherten Bilddaten zur Informationsverarbeitungsvorrichtung übertragen werden.

18. Programm nach Anspruch 5, wobei das Programm des Weiteren die Informationsverarbeitungsvorrichtung anweist, auszuführen:
einen Schritt zum Auswählen einer Abfolge des Übertragens der in der Vielzahl von Empfängern gespeicherten Bilddaten zur Informationsverarbeitungsvorrichtung.

19. Programm nach Anspruch 5, wobei das Programm des Weiteren die Informationsverarbeitungsvorrichtung anweist, auszuführen:
einen Schritt zum Angeben eines Übertragungszustandes der Bilddaten und/oder einer Übertragungsrate der Bilddaten mittels einer Ziffer und/oder eines Buchstabens und/oder einen Schritt zum Steuern des Empfängers, um den Übertragungszustand der Bilddaten und/oder die Übertragungsrate der Bilddaten mittels einer Ziffer und/oder eines Buchstabens anzugeben.

20. Verfahren, auszuführen durch ein kapselartiges Endoskopsystem, das aufweist:
eine Vielzahl von Empfängern; eine Vielzahl von Anbringeinheiten zum jeweiligen Anbringen der Vielzahl von Empfängern; und die Informationsverarbeitungsvorrichtung, die über die Anbringeinheiten mit der Vielzahl von Empfängern verbunden ist, wobei die Informationsverarbeitungsvorrichtung
ermittelt, dass einer der Empfänger an einer der Anbringeinheiten angebracht ist,
vom ermittelten Empfänger über die Anbringeinheit
Patientenidentifikationsinformationen empfängt, die einen Patienten, welcher ein Beobachtungsziel von Bilddaten eines Beobachtungsbilds vom Innern eines Körpers ist und dessen Körperinneres durch ein kapselartiges Endoskop abgebildet worden ist, identifizieren, wobei die Bilddaten vom kapselartigen Endoskop über eine drahtlose Kommunikation empfangen worden sind und im ermittelten Empfänger gespeichert werden,
Empfängeridentifikationsinformationen, die den ermittelten Empfänger identifizieren, oder Informationen zur Anbringeinheit-Identifikation, welche die ermittelte Anbringeinheit identifizieren, den empfangenen Patientenidentifikationsinformationen zuordnet, um eine Übereinstimmungsbeziehung zwischen ihnen zu speichern,
die Übereinstimmungsbeziehung durch Anzeigen zumindest eines Teils der Patientenidentifikationsinformationen, die auf jedem der Vielzahl von Empfängern angezeigt werde, oder zumindest eines Teils der Patienteninformationen, die einen Patienten betreffen, der den Patientenidentifikationsinformationen zugeordnet ist, und die auf jedem der Vielzahl von Empfängern angezeigt werden, anzeigt,
die vom ermittelten Empfänger über die Anbringeinheit übertragenen Bilddaten empfängt, und
einen Fortschritt der Übertragung und/oder eine Übertragungsrate der Übertragung angezeigt.

## Revendications

1. Système d'endoscope de type capsule, comprenant: une pluralité de récepteurs destinés à recevoir, depuis un endoscope de type capsule par le biais d'une communication sans fil, des données d'images d'une image d'observation d'un intérieur d'un corps d'un candidat imagé par l'endoscope de type capsule; une pluralité d'unités de montage destinées à monter respectivement la pluralité de récepteurs; et un appareil de traitement d'informations connecté à la pluralité de récepteurs par le biais des unités de montage, dans lequel
chacun parmi la pluralité de récepteurs comprend une première unité de mémoire et une première unité d'affichage,
la première unité de mémoire mémorise des informations d'identification du candidat identifiant le candidat et les données d'images reçues,
la première unité d'affichage affiche les informations d'identification du candidat ou des informations de candidat concernant le candidat qui sont associées aux informations d'identification de candidat,
l'appareil de traitement d'informations comprend:
une unité de gestion destinée à recevoir les informations d'identification de candidat de chacun parmi la pluralité de récepteurs par le biais de l'unité de montage et destinée à gérer une relation de correspondance entre les informations d'identification de récepteur identifiant le récepteur ou les informations d'identification d'unité de montage identifiant l'unité de montage sur laquelle le récepteur est monté et les informations d'identification de candidat reçues;
une deuxième unité de mémoire; et
une deuxième unité d'affichage,
les données d'images sont transmises vers l'appareil de traitement d'informations par le biais de l'unité de montage et sont mémorisées dans la deuxième unité de mémoire,
la deuxième unité d'affichage affiche la relation de correspondance en affichant au moins une partie des informations d'identification de candidat affichées sur la première unité d'affichage ou au moins une partie des informations de candidat affichées sur la première unité d'affichage, et
la deuxième unité d'affichage affiche en outre une progression de la transmission des données d'images correspondant à chacun parmi la pluralité de récepteurs.

2. Système d'endoscope de type capsule selon la revendication 1, dans lequel la pluralité d'unités de montage est une pluralité de fentes prévues au moins au niveau d'un dispositif de berceau.

3. Système d'endoscope de type capsule selon la revendication 1, dans lequel la pluralité d'unités de montage correspond à des fentes prévues au niveau de chacun parmi une pluralité de dispositifs de berceau.

4. Système d'endoscope de type capsule selon la revendication 3, dans lequel les informations d'identification d'unité de montage sont soit des informations d'identification de port identifiant une pluralité de ports qui sont compris dans l'appareil de traitement d'informations et auxquels la pluralité de dispositifs de berceau peuvent respectivement être connectés, soit des informations d'identification de dispositif de berceau identifiant la pluralité de dispositifs de berceau.

5. Programme pour un système d'endoscope de type capsule qui comprend: une pluralité de récepteurs destinés à recevoir, d'un endoscope de type capsule au moyen d'une communication sans fil, des données d'images d'une image d'observation d'un intérieur d'un corps d'un candidat imagé par l'endoscope de type capsule, de façon à mémoriser les données d'images, et destiné à mémoriser des informations d'identification de candidat qui identifient le candidat; une pluralité d'unités de montage permettant que la pluralité de récepteurs soient montés respectivement; et un appareil de traitement d'informations connecté à la pluralité de récepteurs par le biais des unités de montage, dans lequel le programme dirige l'appareil de traitement d'information pour exécuter:
une étape consistant à détecter que l'un quelconque parmi les récepteurs est monté sur l'une quelconque parmi les unités de montage;
une étape consistant à recevoir les informations d'identification de candidat provenant du récepteur détecté par le biais de l'unité de montage;
une étape consistant à associer des informations d'identification de récepteur identifiant le récepteur détecté ou des informations d'identification d'unité de montage identifiant l'unité de montage détectée avec les informations d'identification de candidat reçues, de façon à mémoriser une relation de correspondance entre elles;
une étape consistant à afficher la relation de correspondance en affichant au moins une partie des informations d'identification de candidat affichées sur chacun parmi la pluralité de récepteurs ou au moins une partie des informations de candidat qui concernent un candidat qui est associé aux informations d'identification de candidat et qui sont affichées sur chacun parmi la pluralité de récepteurs; et
une étape consistant à recevoir les données d'images transmises depuis le récepteur détecté par le biais de l'unité de montage tout en affichant au moins soit une progression de la transmission soit une vitesse de transfert de la transmission.

6. Système d'endoscope de type capsule selon la revendication 3, dans lequel au moins l'un parmi la pluralité de dispositifs de berceau comprend un circuit de chargeur destiné à charger le récepteur monté sur la fente alors que le récepteur transmet les données d'images.

7. Système d'endoscope de type capsule selon la revendication 1, dans lequel au moins soit la première unité d'affichage du récepteur soit la deuxième unité d'affichage de l'appareil de traitement d'informations affiche un état de charge du récepteur.

8. Système d'endoscope de type capsule selon la revendication 1, dans lequel le récepteur comprend un dispositif d'éclairage destiné à indiquer un état de transfert des données d'images.

9. Système d'endoscope de type capsule selon la revendication 1, dans lequel au moins soit la première unité d'affichage du récepteur soit la deuxième unité d'affichage de l'appareil de traitement d'informations affiche au moins soit un chiffre soit un caractère indiquant un état de transfert des données d'images.

10. Programme selon la revendication 5, dans lequel le programme dirige en outre le dispositif de traitement d'informations pour exécuter
au moins soit une étape consistant à indiquer un état de charge du récepteur soit une étape consistant à commander le récepteur pour indiquer l'état de charge du récepteur.

11. Programme selon la revendication 5, dans lequel le programme dirige en outre le dispositif de traitement d'informations pour exécuter
au moins soit une étape consistant à indiquer un état de transfert des données d'images au moyen d'au moins soit un chiffre soit un caractère, soit une étape consistant à commander le récepteur pour indiquer l'état de transfert des données d'images au moyen d'au moins soit un chiffre soit un caractère.

12. Système d'endoscope de type capsule selon la revendication 1, comprenant une unité de commande destinée à commander la deuxième unité d'affichage pour afficher des informations correspondant au récepteur tout en changeant au moins l'un parmi une couleur, une brillance et un type de clignotement conformément à la progression de la transmission des données d'images, lorsque les données d'images mémorisées dans la première unité de mémoire de la pluralité de récepteurs sont transmises vers l'appareil de traitement d'informations.

13. Système d'endoscope de type capsule selon la revendication 1, comprenant une unité de commande destinée à sélectionner une séquence consistant à transférer des données d'images mémorisées dans la première unité de mémoire de chacun parmi la pluralité de récepteurs vers l'appareil de traitement d'informations.

14. Système d'endoscope de type capsule selon la revendication 1, comprenant
une unité de commande destinée à sélectionner l'un parmi une pluralité de choix d'un procédé de transfert destiné à transférer chaque morceau des données d'images mémorisées dans la première unité de mémoire de chacun parmi la pluralité de récepteurs vers l'appareil de traitement d'informations, dans lequel
la pluralité de choix comprend au moins l'un parmi
un transfert en série qui réalise, séquentiellement pour chacun parmi la pluralité de récepteurs, un processus destiné à transférer la totalité des données d'images de la première unité de mémoire de l'un des récepteurs à l'appareil de traitement d'informations,
un transfert alternatif qui répète le transfert de chaque partie des données d'images mémorisées dans chaque première unité de mémoire alternativement de la pluralité de récepteurs à l'appareil de traitement d'informations, et
un transfert en parallèle qui transfère simultanément et parallèlement chaque morceau des données d'images de la première unité de mémoire de chacun parmi la pluralité de récepteurs à l'appareil de traitement d'informations.

15. Système d'endoscope de type capsule selon la revendication 1, comprenant une unité de rétention destinée à fixer mécaniquement le récepteur transférant les données d'images afin d'empêcher que le récepteur soit détaché de l'unité de montage.

16. Système d'endoscope de type capsule selon la revendication 1, dans lequel au moins soit la première unité d'affichage du récepteur soit la deuxième unité d'affichage de l'appareil de traitement d'informations indique au moins soit un état de transfert des données d'images soit une vitesse de transfert des données d'images au moyen d'au moins soit un chiffre soit un caractère.

17. Programme selon la revendication 5, dans lequel le programme dirige en outre l'appareil de traitement d'informations pour exécuter
une étape consistant à afficher des informations correspondant au récepteur tout en changeant au moins l'une parmi une couleur, une brillance, et un type de clignotement conformément à la progression de la transmission des données d'images, lorsque les données d'images mémorisées dans la pluralité de récepteurs sont transmises vers l'appareil de traitement d'informations.

18. Programme selon la revendication 5, dans lequel le programme dirige en outre l'appareil de traitement d'informations pour exécuter
une étape consistant à sélectionner une séquence de transfert des données d'images mémorisées dans la pluralité de récepteurs vers l'appareil de traitement d'informations.

19. Programme selon la revendication 5, dans lequel le programme dirige en outre l'appareil de traitement d'informations pour exécuter
au moins soit une étape consistant à indiquer au moins soit un état de transfert des données d'images soit une vitesse de transfert des données d'images au moyen d'au moins soit un chiffre soit un caractère, soit une étape consistant à commander le récepteur pour indiquer au moins soit l'état de transfert des données d'images soit la vitesse de transfert des données d'images au moyen d'au moins soit un chiffre soit un caractère.

20. Procédé devant être exécuté par un système d'endoscope de type capsule qui comprend: une pluralité de récepteurs ; une pluralité d'unités de montage permettant que la pluralité de récepteurs soient respectivement montés; et l'appareil de traitement d'informations connecté à la pluralité de récepteurs par le biais des unités de montage, dans lequel l'appareil de traitement d'informations
détecte que l'un quelconque des récepteurs est monté sur l'une quelconque des unités de montage,
reçoit, du récepteur détecté par le biais de l'unité de montage, des informations d'identification de candidat identifiant un candidat qui est une cible d'observation de données d'images d'une image d'observation d'un intérieur d'un corps et dont le corps a été imagé depuis l'intérieur par un endoscope de type capsule, les données d'images ayant été reçues depuis l'endoscope de type capsule par le biais d'une communication sans fil et étant mémorisées dans le récepteur détecté,
associe les informations d'identification de récepteur identifiant le récepteur détecté ou les informations d'identification d'unité de montage identifiant l'unité de montage détectée avec les informations d'identification de candidat reçues, de façon à mémoriser une relation de correspondance entre elles,
affiche la relation de correspondance en affichant au moins une partie des informations d'identification de candidat affichées sur chacun parmi la pluralité de récepteurs ou au moins une partie des informations de candidat qui concernent un candidat qui sont associées aux informations d'identification de candidat et qui sont affichées sur chacun parmi la pluralité de récepteurs,
reçoit les données d'images transmises depuis le récepteur détecté par le biais de l'unité de montage, et
affiche au moins soit une progression de la transmission soit une vitesse de transfert de la transmission.
